# EUROPEAN PATENT APPLICATION

(11) **EP 1 677 235 A1**
(43) Date of publication of application: **05.07.2006**
(21) Application number: 04747125.5
(22) Date of filing: 07.07.2004
(51) Int. Cl.: G06Q 10/00

(54) **IMAGE DATABASE SYSTEM**

(71) Applicant: Yamatake, Satoshi, Chofu-shi, Tokyo 182007 (JP)
(72) Inventor: Yamatake, Satoshi, Chofu-shi, Tokyo 182007 (JP)
(74) Representative: Betten & Resch
(86) International application number: PCT/JP2004/009656
(87) International publication number: WO 2006/006202

(57) **Abstract**

It is an object of the present invention to provide an image database system capable of easily correlating medical image data with the object part of the medical opinion data interpreted at the time of preparing the medical opinion data and accurately evaluating the diagnosis. A viewer includes: position specification means for specifying any position of the displayed image; report displaying means for displaying a template for inputting opinion data for the position specified by the position specification means so that the position specified by the position specification means is indicated; and report information storing means for storing the opinion or diagnostic data inputted in the template as report information in the image database server. The report information is stored with link information table data associated with the image data corresponding to the inputted opinion or diagnostic data and the specification position data for the image data in the image database server.

## Description

### TITLE OF THE INVENTION

The present invention relates to an image database system for storing a large quantity of medical image information photographed by a modality device such as a CT device and an MRI device as electronic information, and for contributing to diagnosis. More particularly, the present invention relates to an image database system capable of easily correlating medical image data with the object part of the medical opinion data interpreted at the time of preparing the medical opinion data.

### BACKGROUND

The medical image data generated by the modality device and stored in an image server is reproduced on a screen by a viewer provided with a high-resolution display for exclusive use as disclosed in Japanese Unexamined Patent Publication No. 273364 (2001). A diagnostician operates an opinion producing terminal connected to a report server for controlling report data, and inputs opinion data while interpreting the image reproduced on the viewer. Thus, the prepared report data is stored in the report server in the state that the prepared report data is linked through image data and link data which are displayed on the viewer. There is proposed an image database system constituted so that the report and the object image can be simultaneously displayed on the opinion producing terminal, and another database system constituted so that the report data can be displayed so as to synchronize with the report displayed on the opinion producing terminal.

### DISCLOSURE OF THE INVENTION

### PROBLEMS TO BE SOLVED BY THE INVENTION

However, referring to the image database system which displays the report and the object image simultaneously on the opinion producing terminal, a problem exists in that the object image cannot be displayed clearly due to the limited resolution of the terminal and it is difficult to evaluate the justification of diagnosis by visually inspecting the opinion producing terminal. Referring to the database system constituted so that the report data can be displayed so as to synchronize with the report displayed on the opinion producing terminal, a problem exists in that the site of the object image for inputting the opinion data cannot be apparently specified and it is difficult to evaluate the justification of diagnosis similarly.

### MEANS FOR SOLVING THE PROBLEMS

The present invention has been accomplished in view of the foregoing and other problems in prior art. Accordingly, it is an object of the present invention to provide an image database system capable of easily correlating medical image data with the object part of the medical opinion data interpreted at the time of preparing the medical opinion data and accurately evaluating the diagnosis.

An image database system according to the present invention so as to attain the object, comprising:
a storage unit for storing medical image data;
an image database server for storing attribute information including key information associated with the medical image data stored in the storage unit
a viewer for acquiring and displaying the medical image data from the image database server,
the viewer includes:
   position specification means for specifying the any position of the displayed image;
   report displaying means for displaying a template for inputting opinion data to the position specified by the position specification means so that the specified position specified by the position specification means is indicated; and
   report information storing means for storing the opinion or diagnostic data inputted in the template as report information in the image database server,
wherein the report information is stored with link information table data associated with the image data corresponding to the inputted opinion or diagnostic data and the specification position data to the image data in the image database server.

According to the configuration described above, the opinion data can be inputted through the viewer. In addition, since the template of which the position is specified by the report displaying means is displayed by specifying the object part of the image of diagnosis object displayed on the viewer by using the position specification means, the diagnostician can input the opinion data and the diagnostic data without making mistakes. Since the inputted report information is stored in the image database server with the link information table data associated with the image data and the specification position data to the image data, the report is displayed so as to indicate the object position for diagnosis of the image data even when the report data is to be evaluated later, and the evaluation of the diagnosis can be also performed certainly.

The image database system according to claim 1, wherein the link information table data includes the displaying condition of the image data due to the viewer.

The image database system according to claims any one of 1 and 2, wherein the report displaying means includes report summary information display means for displaying report summary information including one or more opinions or diagnostic data to the image data as a diagnostic object, and a thumbnail image of the image data, and the report information storing means adds diagnostic level data capable of identifying primary registration data due to a primary diagnostician, and definite registration data due to a secondary diagnostician to the report summary information, and stores the data in the image database server.

According to the configuration described above, a result for each inspection unit can be controlled as the report summary information and the diagnostic level can be controlled by the registration data. Therefore, all information from both diagnosticians and a family doctor can be easily disclosed and controlled. For example, the data is used for controlling the access right, a family doctor is able to access the settled report summary, and as for the report summary leading up to the primary registration, the family doctor is not able to but an image diagnostician who is in charge and a responsible image diagnostician are able to access.

### ADVANTAGEOUS EFFECT OF THE INVENTION

As described above, the present invention can provide the image database system capable of easily correlating medical image data with the object part of the medical opinion data interpreted at the time of preparing the medical opinion data and accurately evaluating the diagnosis.

### BEST MODE FOR CARRYING OUT THE INVENTION

Referring to the drawings, the image database system according to the present invention will be described below.

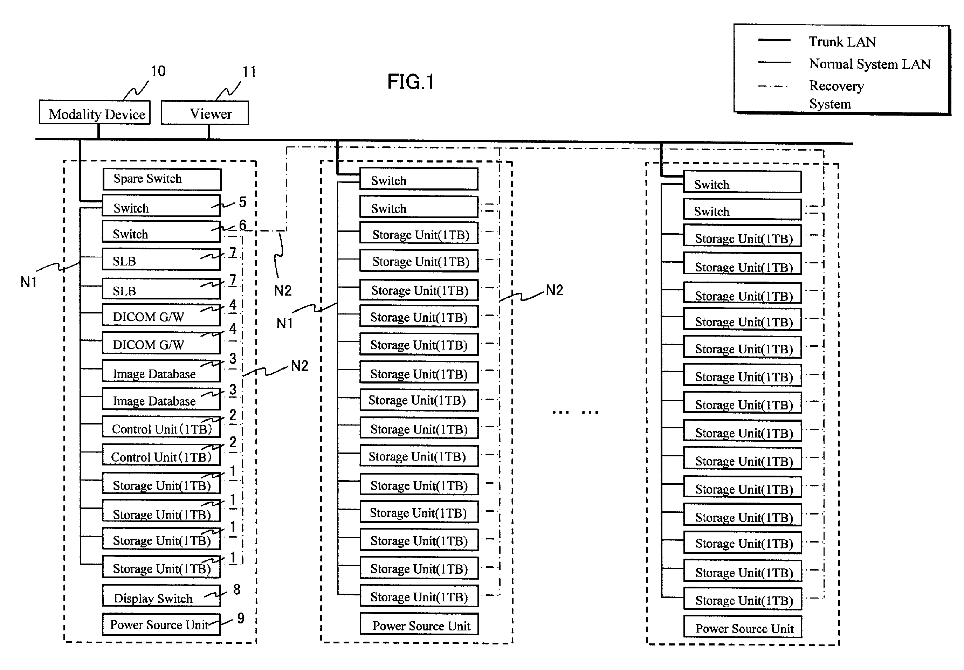

As shown in Fig. 1, the image database system comprises a basic frame (basic minimum tower) and a plurality of extended frames (expanded tower).

The basic frame is constituted by subjecting a storage unit group, a control unit group, an image database server group and a DICOM gateway group with each other to network connection through switches 5 and 6. The storage unit group contains four storage units 1 for storing medical image data. The control unit group contains two control units 2 for controlling each storage unit 1. The image database server group contains two image database servers 3 for storing attribute information including key information associated with the medical image data stored in each storage unit 1 and for applying relay processing of the medical image data between an externally connected viewer 11 and the image database server. The DICOM gateway group contains two DICOM gateways 4 for applying relay processing of the medical image data via a DICOM protocol between a plurality of modality devices 10 externally connected to the image database server 3 and the DICOM gateways. Two load balancers 7 for executing load distribution control of every group is provided for every group based on header information of a request through the network, and a power source unit 8 and a display switch 9 are provided. Herein, the number of units or servers should be a mere exemplar, and the invention is not limited thereto.

The extended frame comprises a power source unit, a plurality of storage units and switches. The extended frame can be extended so that image data with a high capacity can be stored.

The storage units 1, the control units 2, the image database servers 3 and the DICOM gateways 4 are subjected with each other to network connection by a first network N1 through a first switch 5 for processing a job related to an external request from the viewer 11 or the modality device 10, and a second network N2 through a second switch 6 for processing a job related to an internal request. In the networks N1 and N2, a physical layer and data link layer of the network are constituted by Ethernet (registered trademark) of 1000 BASE-T in an OSI reference standard model, and a transport layer and a network layer are constituted by a TCP/IP. High order layers of a session layer or more are operated by a DICOM protocol which is an image diagnostic standard in a medical field, or another local high order protocol.

Two load balancers 7 are connected to each other by a local communication line (RS232-C), and one load balancer is usually operated. For example, load distribution control is executed so that the load of each component may become equivalent by a round-robin system if there is no difference in the performance of the component of each group, a weighted round-robin system if there is a difference in the performance, and a response time algorithm or the like. When a failure is detected through the local communication line, a spare load balancer is operated. Herein, it is also possible to adopt the communication line by another telecommunication standard other than RS232-C as the local communication line. In addition, the local communication may be performed through the first or the second network.

The operation of the system will be explained below by using the response operation or the like to the image storage request, the image read request from the modality device, and the image inspection request from the viewer as an example.

The modality device 10 transfers the image storage request via the DICOM protocol at a virtual IP address (VIP) assigned to the group constituted by two DICOM gateways 4, and starts an association. The load balancer 7 operates normally, and selects the DICOM gateway 4 having the least load. The load balancer 7 intercepts a packet having the virtual IP address as a destination address, and rewrites it to the real IP address of the DICOM gateway 4 selected. The image storage request is received by the selected DICOM gateway 4 according to the above process. The communication of returning packet is established by operating reversely. Herein, the load of the DICOM gateway 4 is distributed in one association by the load balancer 7.

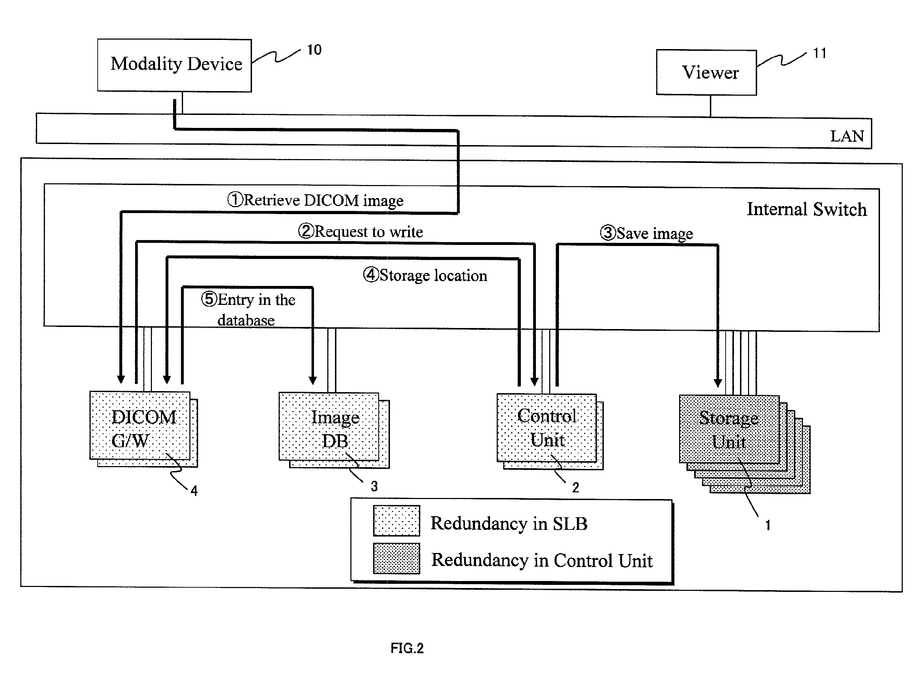

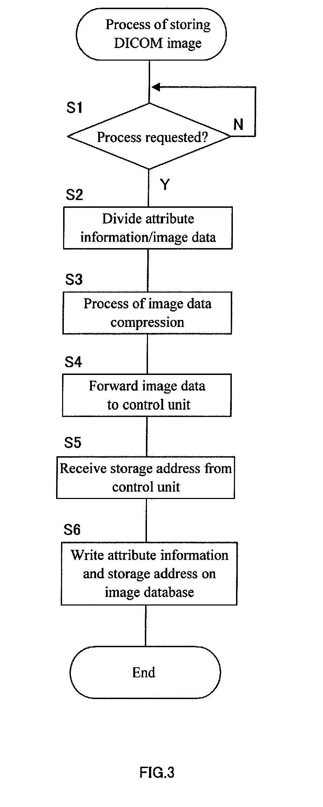

As shown in Figs. 2 and 3, the DICOM gateway 4 requested divides the received image information into the attribute information and image information containing an inspection example UID, the name of a patient, a patient ID, an acceptance number, inspection date, inspection time, and the date of birth of a patient. The inspection example UID consists of a distribution source fronting code, a product specific number and a serial number or the like. The divided image data are compressed into a plurality of images each of having different compression ratios by the DICOM gateway 4 and reversible compression process at a predetermined algorithm is given to each of those images. The compressed images are transferred to the control unit 2. Herein, though four kinds of images (an original image having 512 x 512 pixels, a 1/4 image having 256 x 256 pixels, a 1/16 image having 128 x 128 pixels, and a 1/64 image having 64 x 64 pixels used as a thumbnail) are generated and stored, the compressed images are not limited thereto. Though PNG (Portable Network Graphics) is used, the other reversible compression algorithm may be used.

The control unit 2 receives the image data transferred. The control unit 2 stores the image data with the algorithm to be described below, and replies the storage address to the DICOM gateway 4. The DICOM gateway 4 transfers the attribute information and the storage address of the image data replied from the control unit 2 to the image database server 3, and directs the update processing of data.

The control unit 2 and the image database server 3 involved in the above operation are communicated through the first network N1 in the same manner as the DICOM gateway 4, and the load is distributed by the load balancer 7. Though the load of each operation to be explained below is fundamentally distributed by the load balancer 7 as described above, the explanation of the operation of the load balancer is omitted afterward.

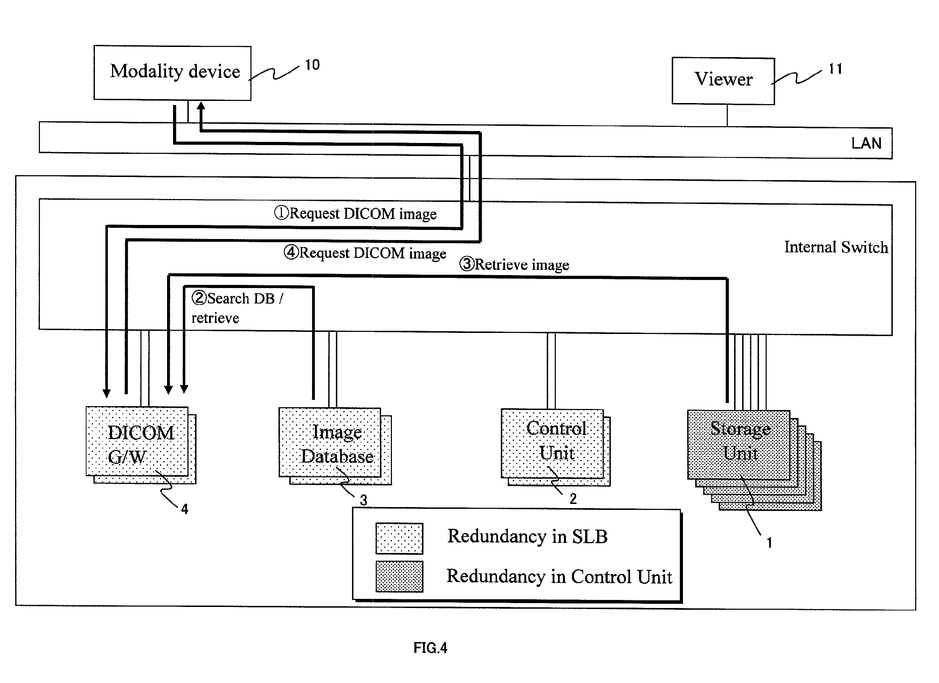

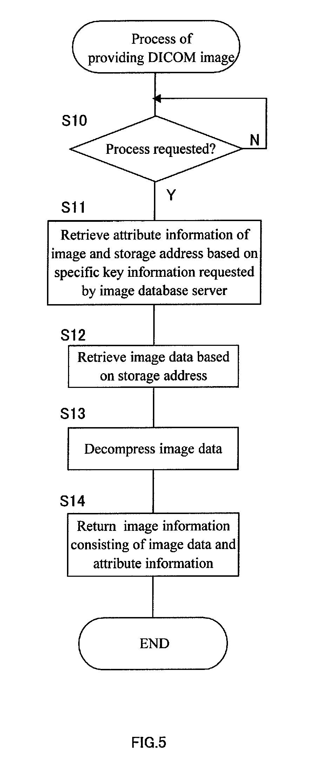

Next, when the DICOM gateway 4 receives an image providing request from the modality device 10, as shown in Figs. 4 and 5, the DICOM gateway 4 retrieves the attribute information and the storage address from the image database server 3 based on any key information of the attribute information specifying a request image. The DICOM gateway 4 acquires the compressed image reversibly compressed from the storage unit 1 based on the storage address, decompresses the compressed image, and replies with the attribute information to the modality device 10 via the DICOM protocol.

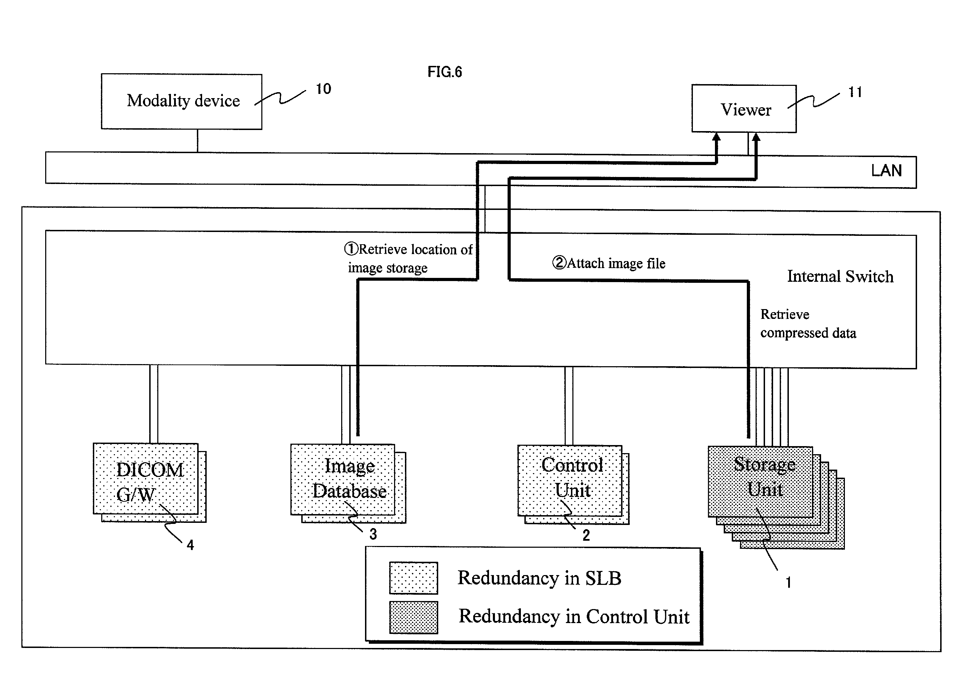

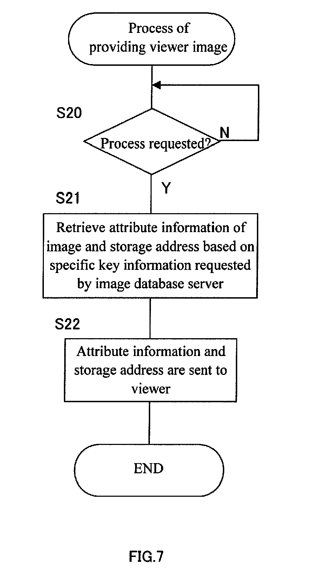

As shown in Figs. 6 and 7, the DICOM gateway 4 acquires the attribute information and the storage address from the image database server 3 based on any key information of the attribute information specifying the requested image according to the inspection request of the image data from the viewer 11 to the image database server 3, and replies to the viewer 11. Then, the viewer 11 accesses the storage unit 1 based on the storage address received, and retrieves a desired image data. Herein, if the viewer 11 is the DICOM viewer, the viewer 11 is operated through the above DICOM gateway 4. However, if the DICOM protocol is not used, and the local protocol by TCP/IP is supported, any high order protocol can be used.

The control unit 2 is provided with operating state inspection means and failure diagnosis means. The operating state inspection means performs an operating state inspection in order through the second network N2 to each storage unit 1 without through the load balancer 7 when a power source is turned on (The load of the external access to the control unit 2 is distributed by the load balancer 7.). Then, the operating state inspection means updates and stores the result in the table showing the operating state of the image database server 3. The failure diagnosis means performs a failure diagnosis of the other control unit based on the update history of the table showing the operating state data due to the other control unit 2.

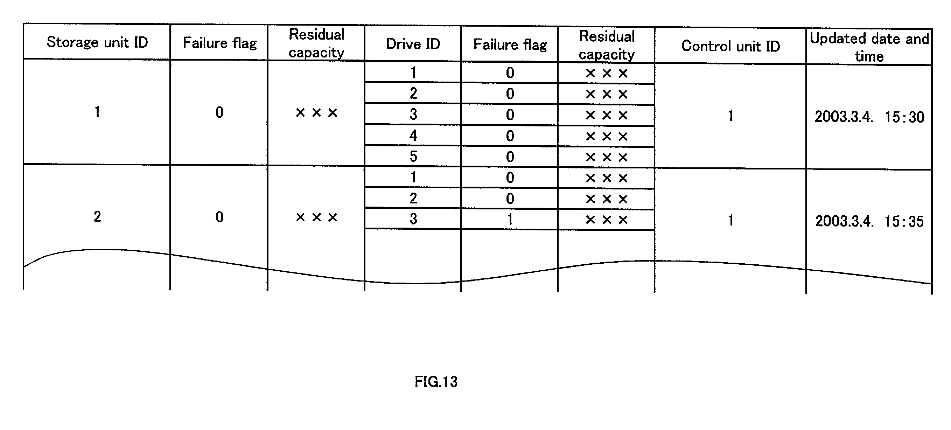

To be detailed below, the operating state inspection means check and see the attachment status of a storage unit in order in a frame unit, whether the unit is removed or attached, in the storage unit 1 which consists of several hard disks and is loaded on the frame, and in a case which the unit is attached, the disk residual capacity for every drive and the total residual capacity of the unit are given. In addition, the operating state inspection means performs the writing and reading of any file to each drive, and investigates the disk obstacle of each drive. The operating state inspection means updates and stores the result at the table showing the operating state of the database server 3 shown in Fig. 13. Though not apparently written in Fig. 13, the data can be controlled in the frame unit by adding a frame ID to the high order data of the storage unit ID.

The control unit 2 is provided with redundancy storing means for selecting data storage locations of the number of redundancy from the storage units which are identified based on the table showing the operating state and are normally operated to the data storage request to the storage unit 1, and for storing the data.

To be detailed below, the redundancy storing means investigates the disk residual capacity per drive based on the table showing the operating state, selects the drives of the number of redundancy in which the disk residual capacity is large to a different storage unit, and stores. Herein, the number of redundancy is set to 2. Therefore, the same data will certainly exist in the different storage unit 1 within the system. (Herein, it is needless to say that the number of redundancy may be set to 2 or more.)

Another method can investigate the disk residual capacity per the storage unit 1, and select two storage units 1 in which the disk residual capacity is large. In addition, the method can select a drive in which the disk residual capacity is large from the storage units 1 selected, and stores the data. The storage address of the data is replied to the transferring origin of a storage request. A reply to the storage request of the above image data is sent to the DICOM gateway 4, and is stored in the database server 3 with the attribute information of the image data or as the attribute information by the DICOM gateway 4.

According to another method, the redundancy storing means may select a plurality of storage units 1 having the residual capacity of a predetermined capacity or more as a candidate based on the table showing the operating state, and select two storage units 1 as an object based on a random number generated by random number generation means. For example, the storage unit of more number than the number of redundancy is selected as the candidate, and any random number of 1 to 9 is generated in order by the random number generation means generates to assign the random number to the storage unit of each candidate. The storage units of the number of redundancy can be selected from the one having the large value of the generated random number. In this case, the load concentrating on the newly added storage unit can be prevented.

Further, according to another method, the redundancy storing means may perform redundancy storage processing by using any method described above to the storage unit mounted on a different frame based on the table showing the operating state. In this case, the influence cased by disasters such as fire, earthquake and flood can be suppressed by setting each frame in a different place and building.

When the redundancy storing means investigates the disk residual capacity based on the table showing the operating state, one having larger residual capacity than the maximum capacity assumed in the image data stored is searched. Conversely, when the residual capacity is smaller than this value, the data indicating the filled drive is stored in the residual capacity data of the table showing the operating state. When all drives of the storage unit 1 are full, the time for investigating the disk residual capacity can be shortened by excluding the storage unit 1 from the object for selection.

Further, when the storage unit 1 or a drive judged to be unusual by the operating state inspection means exists, the control unit 2 retrieves the data storage address of the other storage unit in which the same data as the data stored in the storage unit or a drive judged to be unusual is stored based on the storage address stored in the database server 3 so as to restore the data. The data stored in the data storage address is selected and reproduced from the storage units 1 which is identified based on the table showing the operating state and is normally operated except the storage unit 1 in which the same data is stored. Thus, even if any storage unit breaks down, or is removed, the table showing the operating state to the added storage unit is automatically generated when the image data is automatically reproduced and a new storage unit is added. Thereby, maintenance operation such as the addition and removal of the storage unit can be performed without causing the system down of the power source.

The failure diagnosis means of the control unit 2 accesses the table showing the operating state periodically or irregularly. The failure diagnosis means of the control unit 2 recognizes the failure of the other control unit when the update history does not exist in between the time of access and the past determined time based on the update history of the table showing the operating state data due to the other control units 2. The failure diagnosis means of the control unit 2 starts one spare control unit when the spare control unit is equipped.

Herein, the operating state inspection means may be operated by any one of the two control units under operation, and the failure diagnosis means may be operated by the other control unit. In this case, the flag and operation time data showing the completion of operation of the failure diagnosis means due to the other control unit are recorded on the table showing the operating state, and one control unit checks the flag and the operation time data. Thereby, the failure of the other control unit can be also detected.

The control unit 2 may be constituted integrally with the storage unit 1 on the same substrate. In this case, for example, two storage units are set to an operation state. When one failure is discovered by the failure diagnosis means, the spare control unit can be switched to the operation state.

The update process and failure recovery process of the image database server 3 will be explained below. The image database server 3 is provided with the updating means for updating the database of the image database server 3 to the update processing of data performed through the first network N1 and for updating the database of the other image database server 3 through the second network N2.

The image database server 3 is provided with a port for updating and a port for retrieving to the load balancers 7. Both ports are opened at the normal time. Further, the updating means of one image database server 3 (DB1 in Figure) controls the last operation time of the updating means of the other image database server 3 (DB2 in Figure), and writes in and controls the time of last update on all the records of all tables.

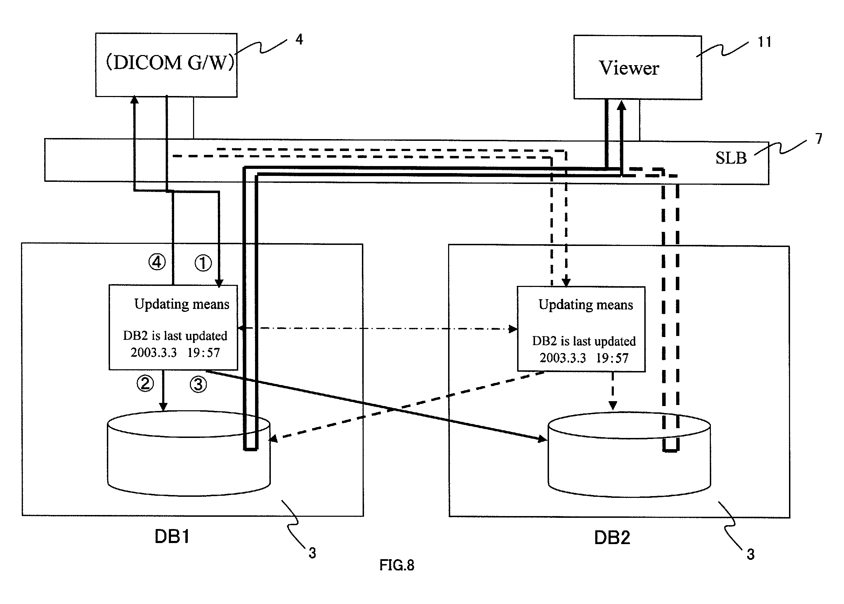

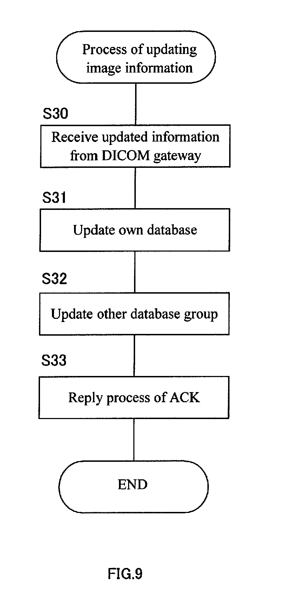

As shown in Figs. 8 and 9, the updating means updates the database (step 2 shown in Fig. 8) according to the data update request such as the storage request of the attribute information of the image data and the storage address of the image data from the DICOM gateway 4 through the load balancer 7, and the storage request of the table showing the operating state from the control unit 2, (step 1 shown in Fig. 8). Then, the updating means updates the database of the other image database server 3 through the second network 2 (step 3 shown in Fig. 8). When this operation is completed, an acknowledgement to the request is sent (step 4 shown in Fig. 8).

The image database server is provided with a port for updating data, and a port for retrieving data to the first network N1. A recovery process means for closing the port for updating data at the time of the recovery process to the database of the other image database server through the second network N2 is provided.

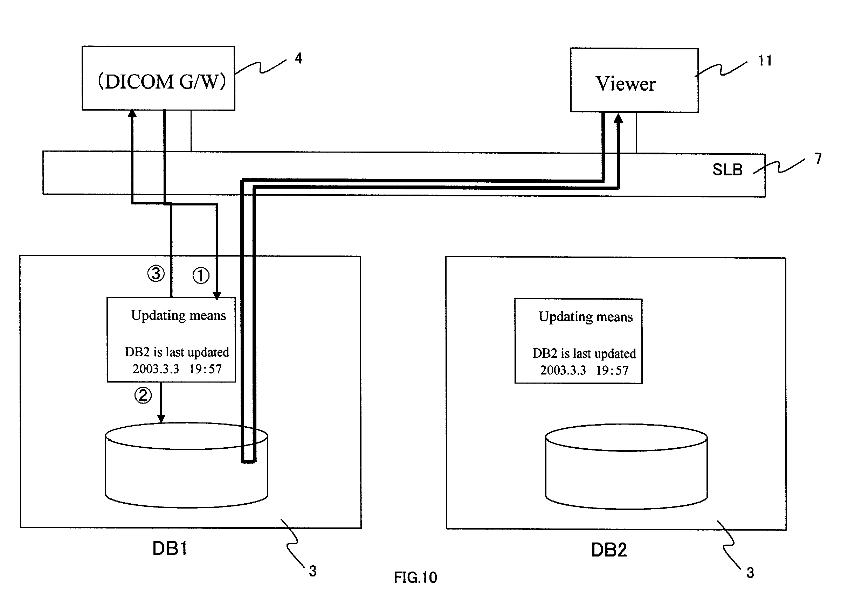

To be detailed below, when the other image database server 3 is out of order, both the port for updating data and the port for retrieving data are closed, and the load balancer 7 requires processing to one of the image database servers 3 only. As shown in Fig. 10, in this case, only the update process of own database is performed to the update request, and an acknowledgement is sent.

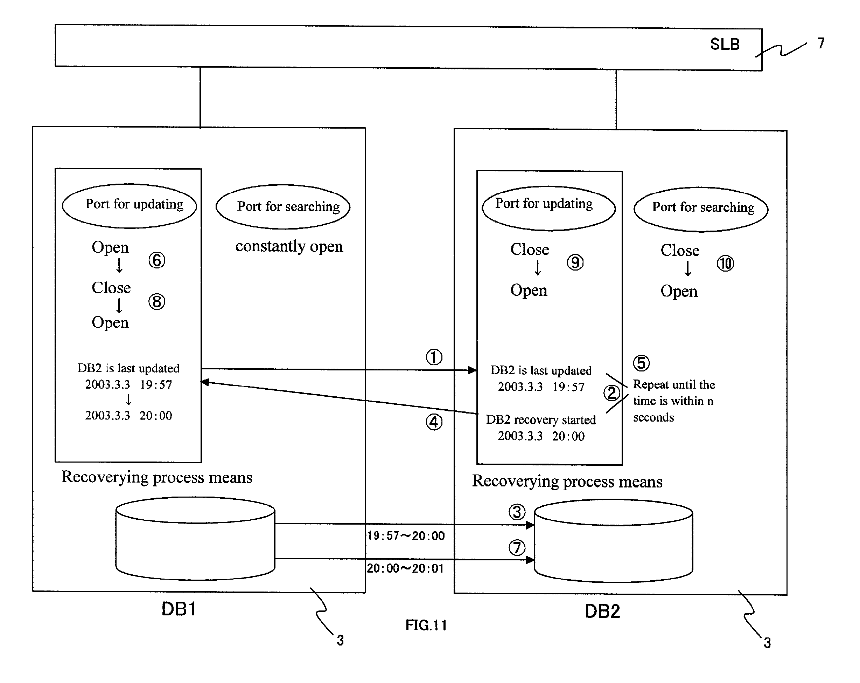

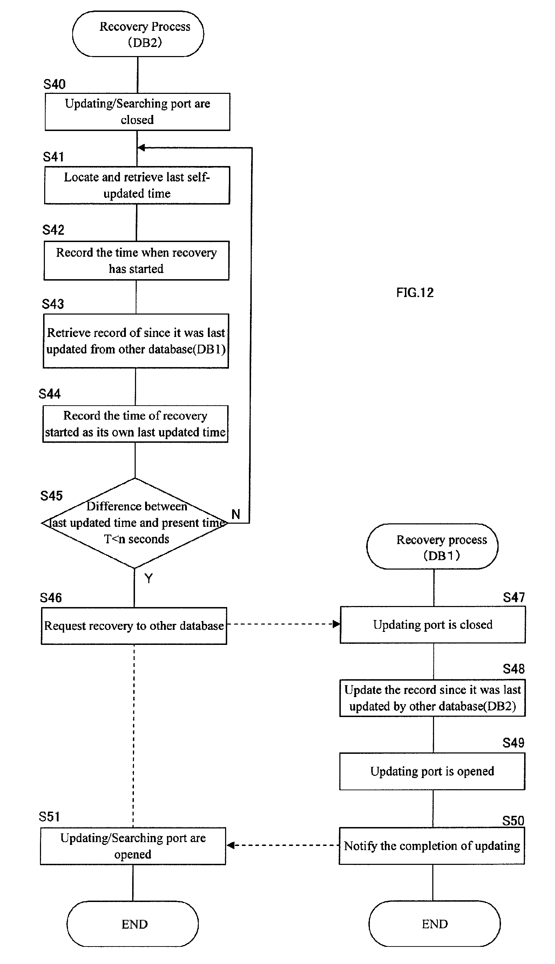

At the time of the restoration from the above failure state, one of the image database servers 3 performs normal operation, and the both ports of the other image database server 3 are closed. As shown in Figs. 11 and 12, the other image database server 3 returning from failure retrieves the last self updated time from one of the image database servers 3 (step 1 shown in Fig. 11), and records restoration start time (step 2 shown in Fig. 11). Next, the record of the updated time after retrieving at the first step is retrieved from one of the image database server 3, and the own database is updated (step 3 shown in Fig. 11). The restoration start time recorded at the step 2 is set to the last updated time of own database (step 4 shown in Fig. 11), the last updated time of the step 4 is compared with the present time. The above steps are repeated until the time is within n seconds. Herein, n is several seconds. If the time is within n seconds, the recovering operation will be requested to one of the image database server 3 (step 5 shown in Fig. 11). On the other hand, one of the image database server 3 closes the port for updating data, and updates the database after the time of the last update of the other image database server 3 (step 6 shown in Fig. 11) (step 7 shown in Fig. 11). The image database server 3 opens the port for updating after completing the update processing (step 8 shown in Fig. 11), and transfers the notice of termination to the other image database server 3. The other image database server 3 opens both the ports, and returns to the normal operation (steps 9, 10 shown in Fig. 11). Though both the port for updating and the port for retrieving are separately constituted in the above explanation, it is also possible to constitute the port for updating and the port for retrieving as the same port.

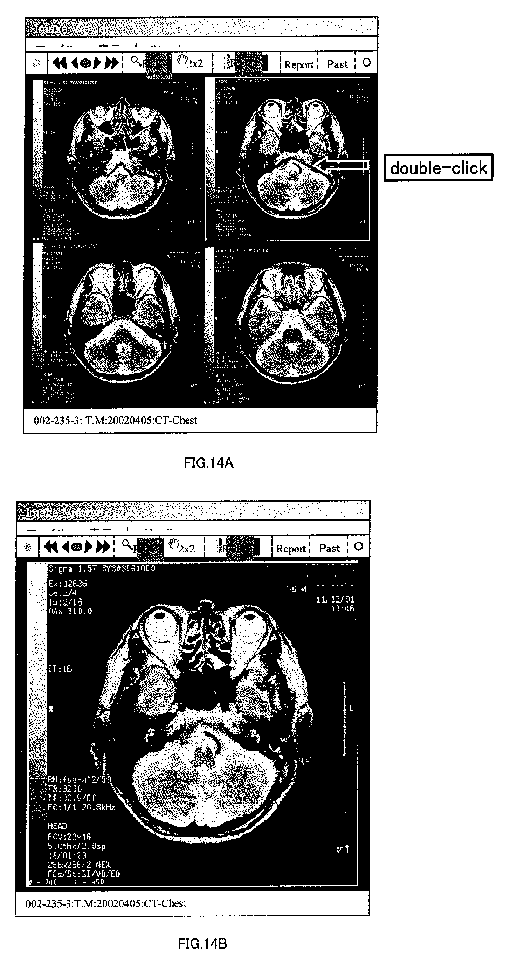

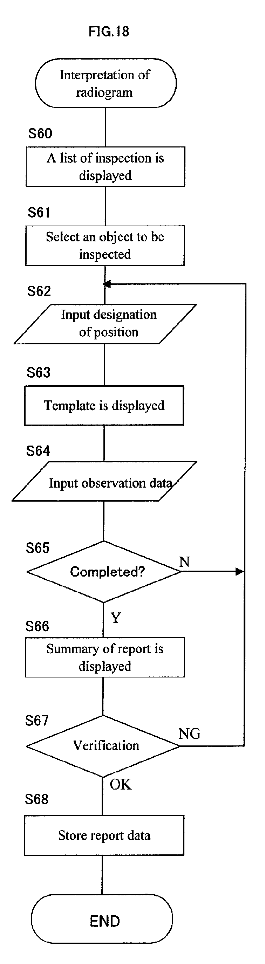

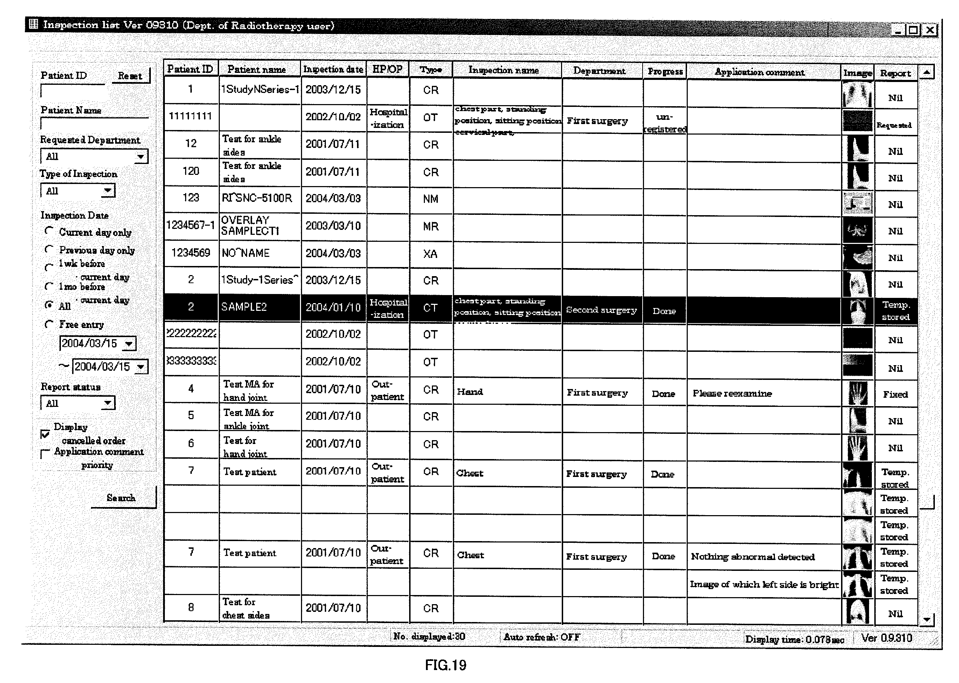

The case where a primary diagnostician interprets for an interpreting processing due to interpreting processing means which is an application program mounted on the viewer 11 will be described below referring to the flow chart shown in Fig. 18. When the interpreting processing means starts up, and a user logs on by inputting a user name and a password in the initial screen, as shown in Fig. 19, the inspection list table stored in the image database server 3 is read (S60), and an object table is displayed by inputting data required for a retrieving key displayed on the left part of the screen, and clicking a retrieving button. When patient specific data (patient ID, name, date of birth, sex or the like) stored in the inspection table, inspection specific data (inspection control number, inspection date, inspection classification, inspection name or the like) and the any area of the image are clicked, for example, a name column for an interpreting object patient is clicked by a mouse (S61), four head tomography images as shown in Fig. 14A are displayed as an initial screen. Whenever a page-updating button is clicked, images are updated and displayed in a unit of four pieces. When the image of which abnormalities are discovered is double-clicked, as shown in Fig. 14B, the enlarged image is displayed.

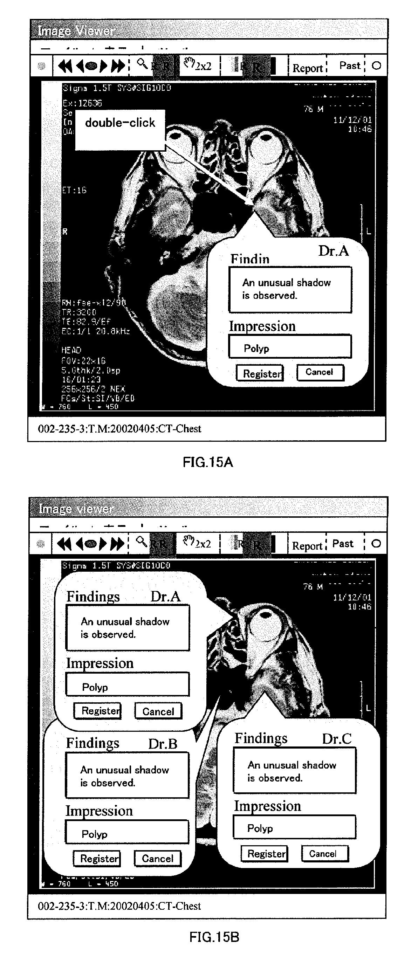

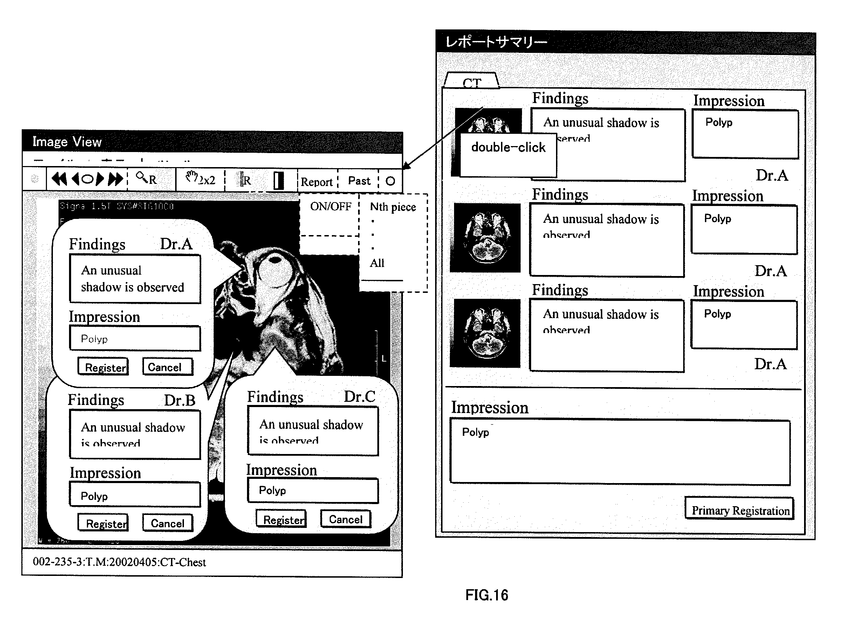

When the abnormality generating part is recognized by carefully examining the image, and the abnormal part is double-clicked by the diagnostician (S62), as shown in Fig. 15A, a template having a blowing shape is displayed so that the position is indicated (S63). The template is provided with columns for describing the opinion data and the diagnostic data. When data is inputted into the column, and a registration button is clicked, the data is primarily stored in storing means embedded in the viewer. When the other abnormalities are discovered in the object image, as shown in Fig. 15B, the process of the step S62 to the step S64 is repeated. When the process is completed (S65), a summary button is clicked from a pull-down menu displayed by clicking a report button of a menu bar, and a report summary image shown at the right hand of Fig. 16 is displayed (S66). Though the shape of the template is not limited to the blowing shape and may be in any shape, it is necessary to indicate the specified position.

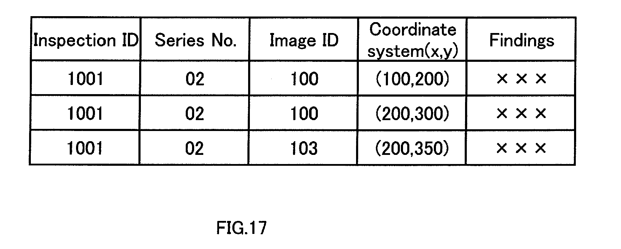

When a diagnostic result is inputted into a diagnostic summary, and the contents are confirmed by a doctor (S67), a primary registration button is clicked, and report information is stored in image database server 3 with a link information table data associated with the image data corresponding to the inputted opinion or diagnostic data and the specification position data to the image data (S68). The link information table data comprises an inspection ID for specifying inspection, a control number of an inspection image due to a modality device, an image ID for specifying the image which becomes an diagnostic object in the inspection image, X and Y coordinate data for showing the position as the object of the report, and the control data (Findings) of the blowing image, as shown in Fig. 17. The image data, the report data and the position data in the image are uniquely linked by the link information table data.

Herein, data such as window width, level, magnifying rate, indicating coordinate and gradation property is preferably added to the link information table data so that the image display condition of the object image displayed on the viewer at the time of inputting the opinion can also be controlled at the same time. Thereby, the doctor referring to the opinion data can reproduce and display on the same indicating condition as the image display condition selected by the diagnostician at the time of interpreting.

That is, the report displaying means for displaying the template for inputting the opinion data so that the specified position due to the position specification means is indicated is constituted by the part executing processing of the step S63 to the step S66. The position specification means for specifying the any position of the displayed image is constituted by the part corresponding to the inputting processing of the position in the step S62. The report information storing means for storing the opinion or diagnostic data inputted into the template as report information in the image database server 3 is constituted by the part executing processing of the step S68.

As shown in Fig. 16, the report displaying means is provided with a report summary information display means for displaying the report summary information consisting of one or more opinions or diagnostic data to the image data which is composed by the part for executing the processing of the step S66 and the diagnostic object, or the thumbnail image of the image data. When the thumbnail image displayed on the report summary is double-clicked, the report displaying means is constituted so that the full image as the diagnostic object is displayed.

Further, the report information storing means is constituted so that the diagnostic level data capable of identifying the primary registration data due to the primary diagnostician and the definite registration data due to the secondary diagnostician is added to the report summary information, and thereby the diagnostic level can be controlled. Specifically, a button shown in the lower right of Fig. 16 is a primary registration button. When the button is clicked, the data is stored as primary registration data. When a diagnosis curator displays the report summary image registered primarily on the viewer, a primary registration button is switched to a secondary registration button. The data is used for the access control of the report information. For example, the data is used for controlling the access right, a family doctor is able to access the settled report summary, and as for the report summary leading up to the primary registration, the family doctor is not able to but an image diagnostician who is in charge and a responsible image diagnostician are able to access.

As explained above, the above image database system is provided with a function as a medical image storing communication system PACS (Image Archive & Communication System) for storing the image data inputted from the modality device 10 through the DICOM gateway 4, and the attribute information (tag information) of the image data such as an inspection example UID, patient attributes (the name of a patient, a patient ID, and the date of birth of a patient or the like), and inspection attributes (an acceptance number, inspection date, inspection time or the like) or the like, and another function as a report information database for storing the report information which is the diagnostic result.

The above inspection list table is generated by table generating means for using each data of patient ID, types of inspection, and inspection date which are included in order information such as an order No. patient ID, types of inspection, and inspection date, and the patient ID, types of inspection and inspection date of the attribute information of the image data inputted from the modality device 10 obtained from Hospital Information System HIS connected to the first network N1 or Radiology information system RIS as a key, and by linking the order No., the HIS information controlled by the order No., and the image data. Thereby, the order No. and the thumbnail image corresponding are controlled so that the order No. and the thumbnail image are related in the same line of the inspection list table.

When Modality Worklist Management MWM specified by DICOM in the radiology information system RIS is mounted, the order information transferred to the modality device 10 by the Modality Worklist Management MWM is incorporated to the attribute information of the image data and outputted, and thereby the inspection list table is generated by using the order No. contained in the attribute information as key by the table generating means.

Since the HIS information is linked with image data by using the patient ID, types of inspection and the inspection date as a key in the first case, a link attachment may be unable to be uniquely performed when the patient ID, types of inspection and the inspection date are the same, and the image data of the different order No. exists. Since the order No. corresponds to the image data one to one in the latter case, the link attachment is always performed uniquely.

The thumbnail image used for the inspection list table is selected based on a predetermined rule from a plurality of image groups of each inspection unit. For example, in the case of a CT image, the image located at a position passing about 1/3 from a tomography image starting position towards the end position is selected, and the image is automatically selected based on the serial No. of the image data.

The image database server 3 is further provided with access history recording means for recording an access history. A machine name (IP address), a login user name, connection time, a study in instance UID (inspection example UID) or the like are recorded at the time of starting the above viewer 11, i.e., at the time of completing the network connection. A SOP instance UID corresponding in addition to the above items may be also recorded as a log information database at the time of the download of the image data from the viewer 11. Thereby, for example, the access record can be retrieved and displayed on the WEB. The operating state such as a monthly using state can be graphically represented to the monitor based on the log information database.

When the inspection list table (not shown) stored in the image database server 3 is read at the above step S60, or a series of image data concerning the patient for interpreting object are read, as described in Figs. 6 and 7, the inspection request of the image data is outputted from the viewer 11 to the image database server 3. The viewer 11 to which the attribute information and the storage address are replied from the image database server 3 based on any key information of the attribute information for specifying the request image accesses the required storage unit 1 based on the received storage address to acquire the desired image data, and displays the image data on the screen. However, the processing of the viewer 11 is constituted in a multitask structure at this time. In addition, an image acquisition request is transferred to each storage unit 1 in a multithread, and a plurality of images is asynchronously received from a plurality of storage units 1. Thereby, even if the displaying speed is enhanced in the viewer 11, and the number of simultaneous connection increases, the load of the network and storage unit can be distributed.

A work station provided with a modeler function for generating a three dimensional model is connected to the first network N1 from a plurality of tomography image data, and the viewer 11 can acquire and display the image data and the three dimensional model from the storage unit 1 through the work station. In this case, the work station transfers the image acquisition request to each storage unit 1 in the multithread, and a plurality of images is asynchronously received from a plurality of storage units 1.

Though the viewer 11 due to a non-DICOM protocol is explained, the viewer 11 is accessed through the above DICOM gateway at the time of using the DICOM viewer as the viewer. The form of the image database server is not also limited to the one described above, and the image database server is suitably constituted.

### BRIEF DESCRIPTION OF THE DRAWINGS

Fig. 1 is a constitution diagram of an image database system according to the present invention;
Fig. 2 is an illustration of a procedure for storing images in the image database system according to the present invention;
Fig. 3 is a flow chart showing the procedure for storing the images in the image database system according to the present invention;
Fig. 4 is an illustration of a procedure for providing images in the image database system according to the present invention;
Fig. 5 is a flow chart showing the procedure for providing the images in the image database system according to the present invention;
Fig. 6 is an illustration of a procedure for searching images by a viewer connected to the image database system according to the present invention;
Fig. 7 is a flow chart showing the procedure for searching the images by the viewer connected to the image database system according to the present invention;
Fig. 8 is an illustration of a data update process by an image database server.
Fig. 9 is a flow chart showing the data update process by the image database server;
Fig. 10 is an illustration of the data update process by the image database server;
Fig. 11 is an illustration of a data recovery process by the image database server;
Fig. 12 is a flow chart showing the data recovery process by the image database server;
Fig. 13 is an illustration of a table showing an operating state;
Fig. 14A is an illustration of a screen displayed on the viewer:
Fig. 14B is an illustration of the screen displayed on the viewer:
Fig. 15A is an illustration of the screen displayed on the viewer:
Fig. 15B is an illustration of the screen displayed on the viewer:
Fig. 16 is an illustration of the screen displayed on the viewer:
Fig. 17 is an illustration of link information table data:
Fig. 18 is a flow chart for explaining the operation of the viewer: and
Fig. 19 is an illustration of an inspection list table.

### EXPLANATION OF REFERENCES

1: Storage Unit
2: Control unit
3: Image database server
4: image database server
5, 6: Switch
7: Load balancer
10: Modality device
11: Viewer

## Claims

1. An image database system comprising:
a storage unit for storing medical image data;
an image database server for storing attribute information including key information associated with the medical image data stored in the storage unit
a viewer for acquiring and displaying the medical image data from the image database server,
the viewer includes:
position specification means for specifying the any position of the displayed image;
report displaying means for displaying a template for inputting opinion data to the position specified by the position specification means so that the specified position specified by the position specification means is indicated; and
report information storing means for storing the opinion or diagnostic data inputted in the template as report information in the image database server,
wherein the report information is stored with link information table data associated with the image data corresponding to the inputted opinion or diagnostic data and the specification position data to the image data in the image database server.

2. The image database system according to claim 1, wherein the link information table data includes the displaying condition of the image data due to the viewer.

3. The image database system according to claims any one of 1 and 2, wherein the report displaying means includes report summary information display means for displaying report summary information including one or more opinions or diagnostic data to the image data as a diagnostic object, and a thumbnail image of the image data, and the report information storing means adds diagnostic level data capable of identifying primary registration data due to a primary diagnostician, and definite registration data due to a secondary diagnostician to the report summary information, and stores the data in the image database server.
